# EUROPÄISCHE PATENTANMELDUNG

(11) **EP 4 729 944 A2**
(43) Veröffentlichungstag der Anmeldung: **22.04.2026**
(21) Anmeldenummer: 25194926.9
(22) Anmeldetag: 08.08.2025
(51) Int. Cl.: G01N 33/575, C12Q 1/6886

(54) **VERFAHREN ZUR ANALYSE EINES TUMORSPEZIFISCHEN MARKERS**

(30) Priorität: 18.10.2024 DE 102024130370
(71) Anmelder: Snipping GmbH, 12053 Berlin (DE)
(72) Erfinder: Nesheim, Nils Martin, 12053 Berlin (DE)
(74) Vertreter: Meissner Bolte Partnerschaft mbB

(57) **Zusammenfassung**

Die vorliegende Erfindung betrifft ein Verfahren zur Analyse eines tumorspezifischen Markers und die Verwendung eines Isolats aus Ejakulat zum Diagnostizieren und/oder Überwachen eines Prostatakarzinoms und/oder zur Erfolgskontrolle einer Therapie eines Prostatakarzinoms.

## Beschreibung

Die vorliegende Erfindung betrifft ein Verfahren zur Analyse eines tumorspezifischen Markers und die Verwendung eines Isolats aus Ejakulat oder eines Isolats aus Exprimaturin zum Diagnostizieren und/oder Überwachen eines Prostatakarzinoms und/oder zur Erfolgskontrolle einer Therapie eines Prostatakarzinoms.

Prostatakrebs ist bei Männern eine häufige Erkrankung und eine der häufigsten Todesursachen.

Daher wurden bereits Verfahren zum Diagnostizieren und Überwachen eines Prostatakarzinoms entwickelt.

Die aktuell von den Krankenkassen in Deutschland finanzierte klinische Diagnostik des Prostatakarzinoms umfasst den PSA-Wert, Tastuntersuchungen und Stanzbiopsien. Der klinische Nutzen des PSA-Wertes als Surrogat-Parameter ist umstritten (Risiko von Overtreatment). Stanzbiopsien gehen als invasives Verfahren mit erhöhtem Aufwand, eigenen Risiken und Belastungen für den Patienten einher und eignen sich daher schlecht für ein engmaschiges Monitoring des Krankheitsverlaufes. In der Regel wird die Metastasierungsaktivität eines Tumors erst im Rahmen der operativen Tumor-Entfernung retrospektiv festgestellt, während der die benachbarten Lymphknoten entnommen und auf Tumor-Infiltration untersucht werden. Dies ist bei der sog. aktiven Beobachtungsstrategie ("active surveillance") relevant, bei der eine operative Prostata-Entfernung herausgezögert wird, um die Lebensqualität des Patienten zu erhalten. Während dieses Monitoringansatzes fehlen diagnostische Verfahren, um zuverlässig den richtigen Zeitpunkt für einen Wechsel in ein aggressives Therapieschema zu bestimmen. Dadurch ergeben sich Risiken für Over- und Undertreatment (i.e. unnötige Operationen oder zu später Therapiebeginn).

Nichtinvasive Verfahren weisen gegenüber invasiven Verfahren wie Stanzbiopsien offensichtlich Vorteile auf. Daher schlägt die Veröffentlichung "Seminal Plasma as a Source of Prostate Cancer Peptide Biomarker Candidates for Detection of Indolent and Advanced Disease" von Neuhaus J., Schiffer E., von Wilcke P., Bauer HW., Leung H., et al. (2013, PLoS ONE 8(6): e67514. doi:10.1371/journal.pone.0067514) die Verwendung von Plasma aus Ejakulat zur Diagnose des Prostatakarzinoms vor. Das Plasma wird gewonnen, indem die Zellen des Ejakulats, darunter Spermazellen, durch Zentrifugieren entfernt werden. Die vergleichsweise geringe Proteinkonzentration und die Beimischung von anderen Drüsen (z.B. aus dem Nebenhoden) kann hier aber zu einem Verdünnungseffekt führen.

Auch die US 2017 / 0 254 810 A1 befasst sich mit der Analyse von Samenflüssigkeit. Konkret wird ein Verfahren zum Nachweis von Biomarkern für Prostatakrebs offenbart, wobei die Samenflüssigkeit auf Unterformen des Prostataspezifischen Antigens (PSA) untersucht wird.

Die Veröffentlichung "DNA-based detection of prostate cancer in blood, urine, and ejaculates" von GOESSL C. et al. (Annals New York academy of sciences. Circulating nucleic acids in plasma or serum II, Vol. 945, 2001, Nr. 1. S. 51-58. ISSN 0077-8923) befasst sich u.a. mit der Analyse von sedimentierten Zellen aus Exprimaturin.

Die bekannten Verfahren weisen demnach nach wie vor Nachteile auf wie z.B. Risiken für Over- und Undertreatment. Es besteht daher nach wie vor ein Bedarf nach einem verbesserten Verfahren zum Diagnostizieren und/oder Überwachen eines Prostatakarzinoms. Auch besteht ein Bedarf nach einem Verfahren zur Erfolgskontrolle einer Therapie eines Prostatakarzinoms.

Vor diesem Hintergrund besteht eine Aufgabe der vorliegenden Erfindung darin, ein verbessertes Verfahren zur Analyse eines tumorspezifischen Markers und zum Diagnostizieren und/oder Überwachen eines Prostatakarzinoms sowie zur Erfolgskontrolle einer Therapie eines Prostatakarzinoms bereitzustellen.

### Zusammenfassung der Erfindung

Aus den Bestrebungen, diese Aufgabe zu lösen, resultiert in einem ersten Aspekt ein Verfahren zur Analyse eines tumorspezifischen Markers, wobei das Verfahren umfasst: Durchführen einer Analyse an einem Isolat, wobei ein tumorspezifischer Marker ausgewählt aus einem Protein, einer DNA und einer RNA analysiert wird, wobei das Isolat durch Fraktionieren von Ejakulat in eine Zellpopulation geringer Dichte und in eine Zellpopulation hoher Dichte erhalten wurde, wobei die Zellpopulation hoher Dichte, welche reife Spermienzellen umfasst, abgetrennt wurde und das Isolat die Zellpopulation geringer Dichte umfasst.

Aus den Bestrebungen, die eingangs genannte Aufgabe zu lösen, resultiert in einem zweiten Aspekt ein Verfahren zur Analyse eines tumorspezifischen Markers, wobei das Verfahren folgende Schritte umfasst:
1) Fraktionieren von Ejakulat in eine Zellpopulation geringer Dichte und in eine Zellpopulation hoher Dichte, wobei die Zellpopulation hoher Dichte, welche reife Spermienzellen umfasst, abgetrennt wird und ein Isolat erhalten wird, das die Zellpopulation geringer Dichte umfasst,
2) Durchführen einer Analyse an dem Isolat, wobei ein tumorspezifischer Marker ausgewählt aus einem Protein, einer DNA und einer RNA analysiert wird.

Der erste und der zweite Aspekt unterscheiden sich darin, dass das Verfahren gemäß zweitem Aspekt zur Bildung des Isolats das Fraktionieren von Ejakulat umfasst, während das Isolat im Verfahren gemäß erstem Aspekt der Ausgangspunkt ist.

Aus den Bestrebungen, die eingangs genannte Aufgabe zu lösen, resultiert in einem dritten Aspekt ein Verfahren zur Analyse eines tumorspezifischen Markers, wobei das Verfahren umfasst: Durchführen einer Analyse an einem Isolat, wobei ein tumorspezifischer Marker ausgewählt aus einem Protein, einer DNA und einer RNA analysiert wird, wobei das Isolat durch Fraktionieren von Exprimaturin in eine zelluläre Fraktion und in eine zellarme Fraktion erhalten wurde, wobei die zellarme Fraktion abgetrennt wurde und das Isolat die zelluläre Fraktion umfasst.

Aus den Bestrebungen, die eingangs genannte Aufgabe zu lösen, resultiert in einem vierten Aspekt ein Verfahren zur Analyse eines tumorspezifischen Markers, wobei das Verfahren folgende Schritte umfasst:
1) Fraktionieren von Exprimaturin in eine zelluläre Fraktion und in eine zellarme Fraktion, wobei die zellarme Fraktion abgetrennt wird und ein Isolat erhalten wird, das die zelluläre Fraktion umfasst,
2) Durchführen einer Analyse an dem Isolat, wobei ein tumorspezifischer Marker ausgewählt aus einem Protein, einer DNA und einer RNA analysiert wird.

Der dritte und der vierte Aspekt unterscheiden sich darin, dass das Verfahren gemäß viertem Aspekt zur Bildung des Isolats das Fraktionieren von Exprimaturin umfasst, während das Isolat im Verfahren gemäß drittem Aspekt der Ausgangspunkt ist.

Aus den Bestrebungen, die eingangs genannte Aufgabe zu lösen, resultiert in einem fünften Aspekt ein Verfahren zur Analyse eines tumorspezifischen Markers, wobei das Verfahren umfasst:
Durchführen einer Analyse an einem Isolat, wobei ein tumorspezifischer Marker ausgewählt aus einem Protein, einer DNA und einer RNA analysiert wird, wobei das Isolat durch Fraktionieren von Ejakulat einer vasektomierten Person in ein Isolat einer zellulären Fraktion und in ein Isolat einer zellarmen Fraktion erhalten wurde, wobei die Analyse an dem Isolat der zellulären Fraktion und/oder dem Isolat der zellarmen Fraktion durchgeführt wird.

Aus den Bestrebungen, die eingangs genannte Aufgabe zu lösen, resultiert in einem sechsten Aspekt ein Verfahren zur Analyse eines tumorspezifischen Markers, wobei das Verfahren folgende Schritte umfasst:
1) Fraktionieren von Ejakulat einer vasektomierten Person in eine zelluläre Fraktion und in eine zellarme Fraktion, wobei aus Ejakulat einer vasektomierten Person ein Isolat der zellulären Fraktion und ein Isolat der zellarmen Fraktion gebildet wird,
2) Durchführen einer Analyse an dem Isolat der zellulären Fraktion und/oder dem Isolat der zellarmen Fraktion, wobei ein tumorspezifischer Marker ausgewählt aus einem Protein, einer DNA und einer RNA analysiert wird.

Der fünfte und der sechste Aspekt unterscheiden sich darin, dass das Verfahren gemäß sechstem Aspekt zur Bildung des Isolats das Fraktionieren von Ejakulat einer vasektomierten Person umfasst, während das Isolat im Verfahren gemäß fünftem Aspekt der Ausgangspunkt ist.

Der der Erfindung zu Grunde liegende Gedanke besteht darin, durch die Isolation und Analyse der zellulären Fraktion mit Ausnahme reifer Spermienzellen, die gegebenenfalls abgetrennt werden, aus Sperma oder Exprimaturin eine Aufkonzentrierung krankheitsrelevanter Marker zu erreichen und Marker ohne Aussagekraft (Background) zu eliminieren. Die Isolation von Zellen aus dem Sperma oder Exprimaturin kann die nichtinvasive Flüssigbiopsie damit quasi zu einer Gewebsbiopsie machen. Das Verfahren kann ein engmaschigeres Monitoring des Prostatakarzinoms ohne Biopsieentnahme mit Aussagekraft über Krankheitsfortschritt und Behandlungserfolg ermöglichen. Der unkomplizierte und kosteneffiziente Test kann eine engmaschigere Kontrolle des Krankheitsverlaufs ermöglichen, als momentan in der klinischen Routine etabliert ist. Damit kann Arzt, Labor und Patient ein Instrument an die Hand gegeben werden, welches beim Behandlungsansatz des "Watchful Waiting" für mehr Klarheit sorgt und zeitnah Veränderungen des Metastasierungsrisikos offenbart. Dadurch können vorzeitige und unnötige operative Eingriffe vermieden (sog. "Overtreatment"), aber auch besonders aggressive Tumoren frühzeitig entdeckt und angemessen behandelt werden. Es besteht die Möglichkeit der Entwicklung eines "Pick-up Service" von Proben, sodass die Patienten im Watchful Waiting Ansatz gar nicht erst die Klinik aufsuchen müssen, sondern lediglich bei einer Veränderung des Markerprofils in ihrer Probe zur Follow-up Untersuchung müssen.

Das erfindungsgemäße Verfahren beschreibt eine nichtinvasive Diagnostik des Prostatakarzinoms. Hierzu werden ejakulierte Prostatazellen und Immunzellen aus Spermaproben oder Prostatazellen und Immunzellen aus Exprimaturinproben eines Patienten isoliert und analysiert. Durch die erhöhte Mobilität metastasierender Zellen können sich diese im Prostatadrüsensekret des Spermas oder Exprimaturins wiederfinden. Durch den Einsatz etablierter Tumormarker können Tumorzellen von gutartigen Zellen unterscheiden werden. Da Spermaproben und Exprimaturinproben regelmäßig abgegeben werden können, erlaubt dies ein engmaschiges Monitoring, sodass die Krankheitsprogression in metastasierende Tumorstadien zeitnah entdeckt werden kann. Hierdurch können günstigere Krankheitsverläufe erzielt (Vermeidung von Undertreatment) und Gelder eingespart sowie unerwünschte Therapieeffekte vermieden werden (Vermeidung von Overtreatment). Die Analyse der Immunzellzusammensetzung in Sperma oder Exprimaturin kann über den Immunstatus des Tumors die Entwicklung und Verlaufskontrolle maßgeschneiderter Therapieansätze ermöglichen (personalisierte Medizin).

Spermaproben wurden bislang nicht als optimaler Analyt angesehen, um Aussagen über Vorgänge in der Prostata zu machen, da die Probe von Spermienzellen und deren Vorläuferzellen dominiert wird. Für die Ejakulation kommt es jedoch zu einer regelrechten, physiologischen Aktivierung und Kontraktion der Prostata, wodurch eine bedeutsame Menge Prostatasekret produziert wird, die einen signifikanten Anteil an der Spermaprobe ausmacht (etwa 30 %). Im Vergleich zur Gewinnung von Prostatasekret aus Exprimaturin nach Prostatamassage kann diese erhöhte Abundanz des Drüsensekrets der Prostata eine höhere Signalstärke von analysierten Krankheitsmarkern und eine geringere Variabilität der erzielten Ergebnisse erlauben. Durch das Abtrennen reifer Spermienzellen und die Isolation und Analyse der Zellfraktion geringer Dichte mit somatischen Zellen aus dem Sperma erreicht man ferner eine Aufkonzentrierung krankheitsrelevanter Marker und eliminiert Signale ohne Aussagekraft (Background). Das erfindungsgemäße Verfahren macht die Flüssigbiopsie damit (quasi) zu einer nichtinvasiven Gewebsbiopsie.

Gegenstand der Erfindung ist auch die Verwendung eines Isolats aus Ejakulat, wobei das Isolat durch Fraktionieren von Ejakulat in eine Zellpopulation geringer Dichte und in eine Zellpopulation hoher Dichte erhalten wird, wobei die Zellpopulation hoher Dichte, welche reife Spermienzellen umfasst, abgetrennt wird, und das Isolat die Zellpopulation geringer Dichte umfasst zum Diagnostizieren und/oder Überwachen eines Prostatakarzinoms und/oder zur Erfolgskontrolle einer Therapie eines Prostatakarzinoms.

Offenbart wird auch die Verwendung eines Isolats aus Exprimaturin, wobei das Isolat durch Fraktionieren von Exprimaturin in eine zelluläre Fraktion und in eine zellarme Fraktion erhalten wird, wobei die zellarme Fraktion abgetrennt wird und das Isolat die zelluläre Fraktion umfasst, zum Diagnostizieren und/oder Überwachen eines Prostatakarzinoms und/oder zur Erfolgskontrolle einer Therapie eines Prostatakarzinoms.

Gegenstand der Erfindung ist auch die Verwendung eines Isolats einer zellulären Fraktion und/oder eines Isolats einer zellarmen Fraktion, wobei das Isolat durch Fraktionieren von Ejakulat einer vasektomierten Person in die zelluläre Fraktion und in die zellarme Fraktion erhalten wird, zum Diagnostizieren und/oder Überwachen eines Prostatakarzinoms und/oder zur Erfolgskontrolle einer Therapie eines Prostatakarzinoms.

### Ausführliche Beschreibung der Erfindung

Ausführungsformen innerhalb dieser Schrift beziehen sich auf alle Aspekte der Erfindung und können beliebig miteinander kombiniert werden, sofern sich aus dem Gegenstand und der Beschreibung der Ausführungsformen nicht eindeutig Gegenteiliges ergibt.

Der Begriff "ein" beziehungsweise "eine" ist zu verstehen als "mindestens ein" beziehungsweise "mindestens eine", sofern sich aus dem Kontext nicht eindeutig Gegenteiliges ergibt.

Die Verben "enthalten" und "umfassen" und ihre Konjugationen umfassen auch das Verb "bestehen aus" mit seinen Konjugationen.

In einer bevorzugten Ausführungsform ist das erfindungsgemäße Verfahren ein Verfahren zum Diagnostizieren und/oder Überwachen eines Prostatakarzinoms.

In einer anderen bevorzugten Ausführungsform ist das erfindungsgemäße Verfahren ein Verfahren zur Erfolgskontrolle einer Therapie eines Prostatakarzinoms. Eine entsprechende Therapie umfasst nicht die radikale Prostatektomie, da hiernach keine Ejakulatprobe mehr abgegeben werden kann. Eine entsprechende Therapie eines Prostatakarzinoms kann beispielsweise ausgewählt sein aus einer Bestrahlung, einer Immuntherapie, einer Androgendeprivationstherapie, einer Androgenrezeptor-gerichteten Therapie (insbesondere mit Tocetaxel und/oder Cabazitaxel), einer Chemotherapie mit einer Platinverbindung (insbesondere mit Carboplatin), einer Therapie mit einem systemischen Radiopharmezeutikum (insbesondere mit einem Therapeutikum ausgewählt aus Radium-233, Lutetium-177-PSMA-617), einer Therapie mit einem PARP-Inhibitor (insbesondere mit Olaparib) oder einer Kombination der vorgenannten Therapien. Bei der Bestrahlung kann es sich um eine perkutane Bestrahlungstherapie, eine Brachytherapie oder eine Kombination davon handeln.

Mit anderen Worten, das Verfahren kann ein Verfahren zur Erfolgskontrolle eines Therapieverfahrens vor Entscheidung zur radikalen Prostatektomie sein. Mit nochmals anderen Worten, das Verfahren kann ein Verfahren zur Erfolgskontrolle eines Therapieverfahrens abseits der radikalen Prostatektomie sein.

In einer bevorzugten Ausführungsform ist der tumorspezifische Marker eine DNA, eine RNA oder ein Protein, wobei der tumorspezifische Marker bevorzugt aus prostataspezifischem Membranantigen (PSMA), prostataspezifischem Antigen (PSA), prostataspezifischer saurer Phosphatase (PAP), Prostein (SLC45A3, auch P501S), Homeobox protein Nkx-3.1 (NKX3.1), Pyruvatkinase M2, Tissue Polypeptide Antigen, Thymidinkinase, Phosphatase and tensin homolog (PTEN), Androgen-Rezeptor (AR; inklusive Amplifikationen, Mutationen und Splicevarianten), Östrogen-Rezeptoren (ESR1, ESR2), Keratinen (KRT14, KRT15, KRT18, KRT19), Kallikrein related peptidase 2 (KLK2), Schlafen 11 (SLFN11), Breast cancer type 1 and 2 susceptibility proteins (BRCA1, BRCA2), Plastin3 (PLS3), Aldehyde dehydrogenase 1A1 (ALDH1A1), Epithelial cell adhesion molecule (EPCAM), Vimentin (VIM), Cadherinen (CDH1, CDH2), Synaptophysin (SYP), Chromogranin (CD56) sowie Delta-like protein 3 (DLL3) ausgewählt ist.

In einer bevorzugten Ausführungsform umfasst die Analyse an dem Isolat ein Verfahren ausgewählt aus Transkriptomanalyse, DNA-Sequenzierung, High-performance liquid chromatography (HPLC), Massenspektrometrie (MS), Immunassay-Verfahren, Durchflusszytometrie sowie einem Verfahren, das sich aus einer Kombination der vorstehenden Verfahren ergibt, wobei die Analyse bevorzugt eine HPLC mit nachgeschalteter MS umfasst.

Erfindungsgemäß erfolgt ein Durchführen einer Analyse an einem Isolat, wobei ein tumorspezifischer Marker ausgewählt aus einem Protein, einer DNA und einer RNA analysiert wird. Bevorzugt wird der tumorspezifischer Marker ausgewählt aus einem Protein, einer DNA und einer RNA quantifiziert. In einer anderen Ausführungsform wird nur eine qualitative Analyse durchgeführt, d.h. es wird analysiert, ob ein Marker gefunden wird oder nicht.

In einer bevorzugten Ausführungsform umfasst die Analyse zumindest einen der folgenden Schritte ausgewählt aus i) einem absoluten Quantifizieren des tumorspezifischen Markers in dem Isolat und ii) einem relativen Quantifizieren des tumorspezifischen Markers in dem Isolat bezogen auf die Gesamtmenge des Proteins beziehungsweise der DNA beziehungsweise der RNA in dem Isolat.

Das Isolat enthält Immunzellen. In einer bevorzugten Ausführungsform werden bei der Analyse an dem Isolat die Zusammensetzung und die Differenzierung der Immunzellen analysiert. Bevorzugt wird zumindest ein Marker ausgewählt aus
- Markern für Immunzell-Infiltration, bevorzugt ausgewählt aus CSF1, CXCL8, CXCR4, G-CSF, MCP-1, SDF-1,
- T-Zell-Differenzierungsmarkern, bevorzugt ausgewählt aus CD3, CD4, CD8, CD25, CD28, CD40L, CTLA4, FasL, PD-1,
- Antigen-Präsentations-Markern, bevorzugt ausgewählt aus MHC I, MHC II,
- B-Zell-Differenzierungsmarkern, bevorzugt ausgewählt aus CD19, CD20, CD21, CD40, CD80, CD86,
- Granulozyten-Differenzierungsmarkern, bevorzugt ausgewählt aus CD11b, CD13, CD14, CD15, CD16, CD18, CD31, CD32, CD33, CD66b, CD117, CD123, CD125, CD170, CD193, Fcε-Rezeptoren, Myeloperoxidase,
- Makrophagen-Differenzierungsmarkern, bevorzugt ausgewählt aus ARG1, CCR2, CD9, CD16, CD40, CD68, CD80, CD86, CD115, CD163, CD169, CD206, CD301, CSF1, CSF1R, CX3CR1, Dectin-1, F4/80, Fizz1, Lyve1, MARCO, NOS2, PDGF beta, PDL2, PPARG, TLR2, TLR4, TREM2,
- Inflammatorischen Cytokinen und Chemokinen, bevorzugt ausgewählt aus CCL2, CCL13, CCL18, IFN-γ, IL1a, IL1b, IL6, IL-1, IL-4, IL-5, IL-8, IL-10, IL-12, IL-13, TGF-β, TNF-α,
analysiert. Das Ergebnis kann weitere Aussagen zu Bösartigkeit und Progression eines Tumors sowie zur Immunabwehr eines Patienten erlauben.

In einer bevorzugten Ausführungsform umfasst das Verfahren ein Inkubieren des Isolats mit einem spezifischen Antikörper, wobei der spezifische Antikörper optional masse- (Massenspektrometrie) oder fluorophormarkiert (DurchflussZytometrie) ist. Im Falle einer massenspektrometrischen Analyse kann die Inkubation der Zellen mit einem massemarkierten markerspezifischen (z.B. anti-PSMA oder anti-PAP) Antikörper das Signal des gesuchten Markers auf eine Größe anheben, wo kein Grundrauschen mehr vorhanden ist. Dadurch lässt sich das Signal "demaskierten". Im Fall des Einsatzes einer Durchflusszytometrie bietet sich ein fluorophormarkierter Antikörper an. Es besteht die Möglichkeit, dass ejakulierte Prostatakarzinomzellen in der Spermaprobe nicht als einzelne Zellen vorliegen, sondern als Zellverband aus dem Tumor gelöst und ausgespült werden. Zur Analyse der Zellen über Durchflusszytometrie kann daher ein enzymatischer Verdau des Isolats zur Zellvereinzelung angewandt werden.

Das erfindungsgemäße Verfahren kann auch das Bereitstellen einer Probe von Ejakulat beziehungsweise von Exprimaturin umfassen, beispielweise kann eine Patientenprobe durch medizinisches Personal oder eine Klink bereitgestellt werden.

In einer bevorzugten Ausführungsform umfasst das erfindungsgemäße Verfahren das Abholen einer Probe von Ejakulat oder Exprimaturin an einem privaten Aufenthaltsort, vorzugsweise am Wohnort, einer Person, wobei das Fraktionieren an dem Ejakulat oder dem Exprimaturin der Person durchgeführt wird. Wie beschrieben erlaubt das nichtinvasive Verfahren mit einfacher Probenabgabe, dass ein "Pick-up Service" angeboten wird und Patienten gar nicht erst eine Klinik aufsuchen müssen, sondern lediglich bei einer Veränderung des Markerprofils in ihrer Probe zur Follow-up Untersuchung müssen.

### Weitere Ausführungen betreffend insbesondere den ersten und zweiten Aspekt

Das erfindungsgemäße Verfahren betrifft in erstem und zweitem Aspekt ein Isolat aus Ejakulat. Bei dem Ejakulat handelt es sich bevorzugt um humanes Ejakulat. Vorzugweise stammt das Isolat aus Ejakulat aus nicht eingefrorenem Material. Mit anderen Worten, vorzugweise war das Ejakulat vor dem Fraktionieren nicht eingefroren.

Das Isolat gemäß erstem und zweitem Aspekt wird durch Fraktionieren von Ejakulat in eine Zellpopulation geringer Dichte und in eine Zellpopulation hoher Dichte erhalten beziehungsweise gebildet, wobei die Zellpopulation hoher Dichte, welche reife Spermienzellen umfasst, abgetrennt wird und das Isolat die Zellpopulation geringer Dichte umfasst. Die Zellpopulation geringer Dichte umfasst somatische Zellen und gametische Vorläuferzellen der Spermienzellen. Vorzugsweise umfasst das Fraktionieren ein Zentrifugieren. Das Zentrifugieren erfolgt beispielsweise bei 200 g bis 600 g, z.B. bei etwa 400 g. Beim Zentrifugieren des Ejakulats wird bevorzugt ein Zellpellet aus den reifen Spermienzellen gebildet und abgetrennt. Die verbliebene Zellpopulation enthält die ejakulierten somatischen Zellen und gametische Vorläuferzellen der Spermienzellen, insbesondere Leukozyten, Epithelzellen, Fibroblasten und andere Zellen. Die reifen Spermienzellen haben eine höhere Dichte und können bei erfindungsgemäßer Verfahrensführung abgetrennt werden, insbesondere bilden sie beim Zentrifugieren ein Pellet.

Bevorzugt umfasst das Fraktionieren von Ejakulat ein Zentrifugieren des Ejakulats, wobei die Zellpopulation geringer Dichte aus somatischen Zellen und gametischen Vorläuferzellen geringer Dichte gebildet wird, wobei das Zentrifugieren des Ejakulats zu einer Anreicherung der somatischen Zellen in dem Isolat und einer Abtrennung reifer Spermienzellen führt. Eine Anreicherung kann eine Erhöhung der Zahl somatischer Zellen pro Volumeneinheit des Isolats und/oder eine Erhöhung der Konzentration somatischer Zellen relativ zu anderen Zellen umfassen.

Bevorzugt wird zum Zentrifugieren eine Zentrifugationslösung verwendet, wobei die Zentrifugationslösung eine Dichte aufweist, die zwischen der Dichte reifer Spermienzellen und der Dichte somatischer Zellen (Leukozyten, Epithelzellen, Fibroblasten) liegt. Hierdurch wird das Trennen der Zellpopulation geringer Dichte (mit der somatischen Zellpopulation) und der Zellpopulation hoher Dichte, welche reife Spermienzellen umfasst, unterstützt. Es können auch mehrere Zentrifugationslösungen verwendet werden, die jeweils eine unterschiedliche Dichte aufweisen, wobei mindestens eine der Zentrifugationslösungen eine Dichte aufweist, die zwischen der Dichte von Spermienzellen und der Dichte somatischer Zellen (der Zellpopulation geringer Dichte) liegt. Werden mehrere solcher Zentrifugationslösungen verwendet, erfolgt die Zentrifugation unter Verwendung eines Dichtegradienten, wobei der Dichtegradient eine Trennung der Zellpopulation geringer Dichte und der Zellpopulation hoher Dichte unterstützt. In einer bevorzugten Ausführungsform enthält die Zentrifugationslösung Ethylendiamintetraacetat.

In einer bevorzugten Ausführungsform wird beim Zentrifugieren des Ejakulats ein inerter Farbstoff zur optischen Unterscheidbarkeit der Zellpopulation geringer Dichte und der Zellpopulation hoher Dichte verwendet. Beispielsweise kann beim Zentrifugieren des Ejakulats ein inerter Farbstoff zur optischen Unterscheidbarkeit einer Phase mit der Zellpopulation geringer Dichte und einer Phase mit der Zellpopulation hoher Dichte verwendet werden.

In einer bevorzugten Ausführungsform umfasst das Verfahren nach dem Fraktionieren mittels Zentrifugieren unter Verwendung einer Zentrifugationslösung und vor dem Durchführen der Analyse ein Verdünnen der Zentrifugationslösung und ein Zentrifugieren, wobei ein Zellpellet gebildet wird. Das Zellpellet beinhaltet somatische Zellen, insbesondere Leukozyten, Epithelzellen und Fibroblasten. Beim Verdünnen der Zentrifugationslösung erfolgt insbesondere eine Anpassung der Dichte der Zentrifugationslösung an die Dichte der Zellpopulation geringer Dichte, wodurch beim nachfolgenden Zentrifugieren besonders gut ein Zellpellet gebildet werden kann. Das Zentrifugieren erfolgt beispielsweise bei 600 g bis 2000 g, z.B. bei etwa 1280 g. In einer bevorzugten Ausführungsform wird das Zellpellet für die Analyse mit Paraformaldehyd fixiert.

Die Analyse wird vorzugsweise an einem Zellpellet durchgeführt. Mit anderen Worten, das Isolat ist oder umfasst in bevorzugten Ausführungsformen ein Zellpellet. Ein Zellpellet hat besondere Vorteile, weil es die Zellen, DNA, RNA und Protein in hoher Konzentration enthält und gut gelagert oder für komplexe Analysen versendet werden kann.

In einer bevorzugten Ausführungsform erfolgt vor dem Bereitstellen des Ejakulats eine Prostatamassage. Mit anderen Worten, in einer bevorzugten Ausführungsform stammt das Ejakulat von einer Person, bei der vor Abgabe einer Probe des Ejakulats eine Prostatamassage durchgeführt wurde. Hierdurch kann die Aussagekraft des Verfahrens gesteigert werden.

In einer bevorzugten Ausführungsform wird das Ejakulat vor dem Fraktionieren einer Wärmebehandlung bei mehr als 25 °C unterzogen. Die Wärmebehandlung erfolgt außerhalb des menschlichen Körpers. Die Wärmebehandlung kann beispielsweise in einem Temperaturbereich von mehr als 25 °C bis 40 °C durchgeführt werden, insbesondere bei etwa 37 °C. Die Wärmebehandlung kann beispielsweise über einen Zeitraum von 5 Minuten bis 60 Minuten erfolgen. Die Wärmebehandlung begünstigt die physiologisch vorgesehene Verflüssigung des Ejakulats. Die Wärmebehandlung kann die Probenaufbereitung und Analysenergebnisse verbessern.

### Weitere Ausführungen betreffend insbesondere den dritten und vierten Aspekt

Das Verfahren betrifft in drittem und viertem Aspekt ein Isolat aus Exprimaturin.

Exprimaturin wird auch als Prostata-Massage-Urin bezeichnet. Mit anderen Worten, unmittelbar vor Abgabe einer Urinprobe erfolgte eine Prostatamassage bei der Person, von welcher der Exprimaturin stammt. Bevorzugt ist die Person ein Mensch. Bevorzugt handelt es sich um humanen Exprimaturin.

Das Isolat aus Exprimaturin wird durch Fraktionieren des Exprimaturins in eine zelluläre Fraktion und in eine zellarme Fraktion erhalten, wobei die zellarme Fraktion abgetrennt wurde und ein Isolat aus Exprimaturin gebildet wird, wobei das Isolat die zelluläre Fraktion umfasst. Die Zellen der zellulären Fraktion stammen im Wesentlichen aus dem Prostatasekret. Die zellarme Fraktion umfasst im Wesentlichen den flüssigen und gelösten Teil des Urins sowie des Prostatasekrets. Die zellarme Fraktion ist vorzugsweise eine zellfreie Fraktion.

Vorzugweise stammt das Isolat aus Exprimaturin aus nicht eingefrorenem Material. Mit anderen Worten, vorzugweise war der Exprimaturin vor dem Fraktionieren nicht eingefroren.

Bevorzugt umfasst das Fraktionieren ein Zentrifugieren. Vorzugsweise bildet das Isolat beim Zentrifugieren ein Zellpellet. Das Zentrifugieren erfolgt beispielsweise bei 600 g bis 2000 g, z.B. bei etwa 1280 g. In einer bevorzugten Ausführungsform wird das Zellpellet für die Analyse mit Paraformaldehyd fixiert.

Die Analyse wird vorzugsweise an einem Zellpellet durchgeführt. Mit anderen Worten, das Isolat ist oder umfasst in bevorzugten Ausführungsformen ein Zellpellet. Ein Zellpellet hat besondere Vorteile, weil es die Zellen, DNA, RNA und Protein in hoher Konzentration enthält und gut gelagert oder für komplexe Analysen versendet werden kann.

### Weitere Ausführungen betreffend insbesondere den fünften und sechsten Aspekt

Das erfindungsgemäße Verfahren betrifft in fünftem und sechstem Aspekt ein Isolat aus Ejakulat einer vasektomierten Person, wobei das Isolat durch Fraktionieren des Ejakulats einer vasektomierten Person in eine zelluläre Fraktion und in eine zellarme Fraktion erhalten wurde. Bei der Person handelt es sich bevorzugt um einen Menschen. Bei dem Ejakulat handelt es sich bevorzugt um humanes Ejakulat.

Bei einer Vasektomie wird die Verbindung des Hodens vom sogenannten "Spritzkanal" (engl. "ejaculatory duct") getrennt, sodass bei der Ejakulation keine Spermienzellen oder gametische Vorläuferzellen in die Spermaprobe gelangen können. In den Ausführungsformen gemäß fünftem und sechstem Aspekt (nach Vasektomie) erübrigen sich die Verfahrensschritte zur Abtrennung reifer Spermienzellen aus dem Ejakulat. Darüber hinaus enthält das Ejakulat auch keine gametischen Vorläuferzellen, was die Aussagekraft des Verfahrens weiter verbessern kann.

Vorzugweise stammt das Isolat aus Ejakulat aus nicht eingefrorenem Material. Mit anderen Worten, vorzugweise war das Ejakulat vor dem Fraktionieren nicht eingefroren.

Bevorzugt umfasst das Fraktionieren ein Zentrifugieren. Vorzugsweise bildet das Isolat der zellulären Fraktion beim Zentrifugieren ein Zellpellet. Das Zentrifugieren erfolgt beispielsweise bei 600 g bis 2000 g, z.B. bei etwa 1280 g. In einer bevorzugten Ausführungsform wird das Zellpellet für die Analyse mit Paraformaldehyd fixiert.

Die Analyse des Isolats der zellulären Fraktion wird vorzugsweise an einem Zellpellet durchgeführt. Mit anderen Worten, das Isolat der zellulären Fraktion ist oder umfasst in bevorzugten Ausführungsformen ein Zellpellet. Ein Zellpellet hat besondere Vorteile, weil es die Zellen, DNA, RNA und Protein in hoher Konzentration enthält und gut gelagert oder für komplexe Analysen versendet werden kann.

In einer bevorzugten Ausführungsform des erfindungsgemäßen Verfahrens erfolgt vor dem Bereitstellen des Ejakulats eine Vasektomie.

In einer bevorzugten Ausführungsform erfolgt vor dem Bereitstellen des Ejakulats eine Prostatamassage. Mit anderen Worten, in einer bevorzugten Ausführungsform stammt das Ejakulat von einer Person, bei der vor Abgabe einer Probe des Ejakulats eine Prostatamassage durchgeführt wurde. Hierdurch kann die Aussagekraft des Verfahrens gesteigert werden.

In einer bevorzugten Ausführungsform wird das Ejakulat vor dem Fraktionieren einer Wärmebehandlung bei mehr als 25 °C unterzogen. Die Wärmebehandlung erfolgt außerhalb des menschlichen Körpers. Die Wärmebehandlung kann beispielsweise in einem Temperaturbereich von mehr als 25 °C bis 40 °C durchgeführt werden, insbesondere bei etwa 37 °C. Die Wärmebehandlung kann beispielsweise über einen Zeitraum von 5 Minuten bis 60 Minuten erfolgen. Die Wärmebehandlung begünstigt die physiologisch vorgesehene Verflüssigung des Ejakulats. Die Wärmebehandlung kann die Probenaufbereitung und Analysenergebnisse verbessern.

Nachfolgend wird die Erfindung anhand von Beispielen illustriert, die nicht beschränkend sein sollen.

### Beispiel 1

### a) Probenabgabe

Probenabgabe: Patienten mit Prostatakarzinom (Prostate carcinoma; PCa) geben zu Hause oder alternativ während einer vorgesehenen Routineuntersuchung in der urologischen Ambulanz der behandelnden Klinik eine Ejakulatprobe (Sperma) ab.

Optional - Prostatamassage vor Probenabgabe: Um eine gesteigerte Mobilisierung von Epithelzellen aus den Drüsengängen der Prostata in das Ejakulat zu ermöglichen, kann der Arzt vor der Ejakulatabgabe eine Prostatamassage durchführen. Diese kann im Rahmen der regulär vorgesehenen Tastuntersuchung der Prostata (Digital Rectal Exam; DRE) erfolgen.

### b) Isolation der somatischen Zellen aus der Ejakulatprobe

Im Anschluss werden die somatischen und die gametischen Zellen geringer Dichte aus der Ejakulatprobe gewonnen. Da Flüssigbiopsien vor Fixierung degradieren, ist der beschriebene Schritt zeitsensitiv und wird bevorzugt noch am Ort der Probenabgabe durchgeführt. Die Schritte nach der Probenfixierung sind weniger zeitsensitiv, sodass danach ein Probenversand ins Zentrallabor erfolgen kann.

Physiologisch bedingte Verflüssigung der Ejakulatprobe: Beim Gewinnen der Zellen wird die Ejakulatprobe zunächst für 15 Minuten in einem Wärmeschrank (37°C) inkubiert, wodurch es zur physiologisch vorgesehenen Verflüssigung der Flüssigbiopsie kommt. Die ejakulierten Zellen werden hierdurch aus der Proteinmatrix des Ejakulats befreit, was die Auftrennung der Probe in die unterschiedlichen Zellfraktionen im Folgeschritt erleichtert.

Auftrennung der Probe durch Zentrifugation in einem Dichtegradienten: Die verflüssigte Ejakulatprobe wird auf einen Dichtegradienten aus einem inerten Stoff ohne physiologischen Einfluss auf die Zellphysiologie geladen. Ein Dichtegradient wird klassischerweise über die Schichtung mehrerer Lösungen mit unterschiedlicher Dichte hergestellt, wobei mit der höchsten Dichte begonnen und mit jeder Schicht eine geringere Dichte eingesetzt wird (z.B.: 90%, 70%, 50%, 30% Konzentration). Bei der Schichtung des Gradienten sollten Turbulenzen und eine Durchmischung der entstehenden Phasen möglichst vermieden werden. Die anschließende Zentrifugation passiert bei sehr geringer Geschwindigkeit (~ 400 g), sodass der Gradient erhalten bleibt. Die Zellen folgen der Zentrifugalkraft in Richtung des Gefäßbodens, wobei sie aufgrund der geringen Kräfte in jener Phase stehenbleiben, welche ihrer eigenen Dichte entspricht.

Optional - Wahl einer einheitlichen Dichte der Gradientenlösung: Da es bei dem vorliegenden Verfahren primär um die Abscheidung der Spermienzellen geht und der Test idealerweise transportabel sein und nicht vor Ort vorbereitet werden soll, bietet sich eine Lösung mit einheitlicher Dichte an. Die gewählte Dichte der Lösung liegt in diesem Fall zwischen der Dichte von Spermienzellen und den übrigen Zellen der Probe. Bei einer Zentrifugation der Ejakulatprobe auf dieser Lösung bilden die Spermienzellen ein Pellet (höhere Dichte als die Lösung) und die übrigen Zellen sammeln sich in der Phase zwischen Seminalplasma und Gradientenlösung an (geringere Dichte als die Lösung). Streng genommen handelt es sich nach dieser Modifikation nicht mehr um einen Dichtegradienten im klassischen Sinne.

Kühlung der Verfahrensschritte vor Fixierung: Da die Zellen mit der Verarbeitung aus ihrem biologischen Milieu entfernt werden, sind Veränderungen von ihrem ursprünglichen Zustand in der Prostata zu erwarten. Eine Abkühlung der Probe führt zu einer Verlangsamung dieser unerwünschten Prozesse. Aus diesem Grund kann ab der Entfernung der Probe aus dem Wärmeschrank (37 °C) mit einer gekühlten Lösung (4 °C) gearbeitet werden. Die Reaktionsgefäße des Assays inklusive Dichtegradienten-Lösung können daher vor Durchführung des Tests im Kühlschrank gelagert werden (4 °C). Eine Kühlzentrifuge würde das Ergebnis weiter verbessern, allerdings kann nicht davon ausgegangen werden, dass alle urologischen Ambulanzen über eine Kühlzentrifuge verfügen.

### Optional - Verwendung von Chelatbildnern oder Protease-Inhibitoren in Dichtegradienten-Lösung:

Chelatbildner (z.B. Ethylendiamintetraacetat; EDTA) und Protease-Inhibitoren in der Lösung können die enzymatische Aktivität in der Probe zusätzlich einschränken und dadurch die Degradierung der Probe verlangsamen. Ihre Wirkung kann jedoch nachteilige Effekte auf Folgeschritte des Verfahrens haben (z.B. verminderte Bindungsfähigkeit von Antikörpern, oder Zellvereinzelung). Aus diesem Grund wird unter Umständen von einer Nutzung dieser Reagenzien abgesehen.

Verfahrensprotokoll: Die folgend beschriebene Probenaufbereitung kann über ein Versuchskit durchgeführt werden, welches die Durchführung möglichst intuitiv und fehlerfrei halten soll. Jene Lösungen des Kits, welche vor dem Fixierungsschritt eingesetzt werden, können vor ihrer Verwendung im Kühlschrank gelagert werden (4 °C):
1) Schichten der Ejakulatprobe (~ 2-6 mL) auf 9 mL Histopaque-Lösung (90 % Histopaque^{®}1077, 10 % Phosphate-buffered Saline; PBS) in einem 15 mL Falcon-Tube;
2) Zentrifugieren des Falcon-Tubes für 30 min bei 400 g (Raumtemperatur = RT);
3) Abnehmen von 9 mL des Überstandes aus der Lösungs-Luft Phase von oben nach unten in kreisförmigen Bewegungen; das entstandene Pellet darf nicht verwirbelt werden, um einen Transfer von Spermienzellen zu vermeiden;
4) Überführen des 9 mL Überstandes in ein 50 mL Falcon-Tube mit 41 ml PBS;
5) Mischen der Zellsuspension durch mehrfaches Invertieren des Falcon-Tubes (Endkonzentration: 16 % Histopaque^{®}1077, 84 % PBS);
6) Zentrifugieren des Falcon-Tubes für 30 min bei 1.280 g (RT);
7) Verwerfen des Überstandes, wobei das Pellet nicht trockenfallen darf;
8) Resuspendieren des Zellpellets in 0,5 mL PFA-PBS (4 % Paraformaldehyd; PFA; RT);
9) Inkubieren der Zellsuspension für 15 min zur PFA-Fixierung (RT);
10) Stoppen der Fixierung durch Zugabe von 40 mL PBS (4 °C) und mehrfaches Invertieren des Falcon-Tubes;
11) Zentrifugieren der Probe im Falcon-Tube für 30 min bei 1.280 g (RT)
12) Abnahme der verdünnten PFA-PBS Lösung, Resuspendierung des Zellpellets in 1 mL PBS (4 °C);
13) Versand der Probe (= des Isolats) ins Zentrallabor (4 °C).

### c) Analyse an dem Isolat

Im Zentrallabor werden die fixierten Proben auf PCa-spezifische Tumormarker (PCa-Marker) untersucht.

### Variante 1: Durchflusszytometrie

### Doppelmarkierung der isolierten Zellen gegen die PCa-Marker PSMA und PAP:

Es ist möglich, Formaldehyd-fixierte Zellen für eine Durchflusszytometrie mit Antikörpern zu markieren. Die fixierte Probe wird im Zentrallabor gekühlt gelagert und verarbeitet (4 °C). In diesem Beispiel erfolgt eine Doppelmarkierung der Zellen mit zwei fluoreszenzmarkierten PCa-Markern (in diesem Beispiel: Prostataspezifische Membranantigen, PSMA; Prostataspezifische saure Phosphatase, PAP). Dieses Beispiel ist aber nicht beschränkend und die Verwendung andere PCa-Marker ist möglich. Die Probenaufbereitung erfolgt nach etablierten Protokollen.

Ergebnisse der Durchflusszytometrie: Bei der Datenerhebung über Durchflusszytometrie werden die fluoreszenzmarkierten Zellen durch eine Kapillare mit sehr geringem Durchmesser geleitet. Der Sensor registriert eine Veränderung der Lichtbrechung, wenn er durch eine fluoreszenzmarkierte Zelle passiert wird. Abseits der Detektion von fluoreszenzmarkierten Antikörpern erlaubt Durchflusszytometrie eine Messung von Größe (Forward Scatter; FSC) und Granularität (Side Scatter; SSC) der untersuchten Zellen. Diese Werte für sich gesehen können bereits von prognostischem Wert für das angewendete Verfahren sein. Darüber hinaus können die genannten PCa-Marker mit fluoreszenzmarkierten Antikörpern nachgewiesen werden. Durch die Relation mehrerer Signale (z.B. FSC, SSC und Fluoreszenzintensität) zueinander lassen sich bei der nachfolgenden Auswertung des Datensatzes definierte Zellpopulationen voneinander zählen und abgrenzen. Durch den Abgleich der für die getesteten Antigene positiv gewerteten Zellen mit der Gesamtzahl der gemessenen Zellen ergibt sich ein Prozentwert für die Probe. Ergebnisbeispiel:
1) 2% der Zellen in der Probe sind PAP-positiv (PAP+);
2) 5% der Zellen in der Probe sind PSMA-positiv (PSMA+);
3) 1,5% der Zellen in der Probe sind sowohl PAP-, als auch PSMA-positiv (PAP+ PSMA+).

Durch die Erfassung des Prozentsatzes von PAP+ und PSMA+ Zellen in der Probe erhält man eine Momentaufnahme über jene mobilen Prostatakarzinomzellen, welche aus dem lokalen Tumor gelöst und in das Ejakulat des Patienten gespült wurden. Dies repräsentiert hochmobile Tumorzellen, welche nicht nur das Ejakulat, sondern auch die benachbarten Lymphknoten und Organe des Patienten infiltrieren können. Aus diesem Grund erlaubt das vorliegende Verfahren eine Aussage über das Metastasierungsrisiko für den Patienten.

### Variante 2: Proteomanalyse der isolierten Zellen

HPLC-MS an fixierten Pellets isolierter somatischer Zellen: Das isolierte, fixierte Zellpellet wird nach etablierten Protokollen aufbereitet und über eine Flüssigchromatographie (High-performance liquid chromatography; HPLC) mit nachgeschalteter Massenspektrometrie (MS) analysiert. Die HPLC erlaubt eine Auftrennung in verschiedene Protein-Fraktionen, welche über die nachgeschaltete MS analysiert werden. Das Verfahren ist etabliert, kommerziell verfügbar und erlaubt bei guter Qualität des Ausgangsmaterials ein Proteinspektrum mit einem Auflösungsvermögen von über 4000 unterschiedlichen Proteinen.

Optional - Markierung der Probe mit goldassoziierten Antikörpern: Das Proteom einer Probe besteht aus hoch abundanten Proteinen und wenig abundanten Proteinen, wodurch sich Probleme bei der Proteomanalyse ergeben können. Die Signale der PCa-Marker können bei geringer Abundanz vom Grundrauschen der hoch abundanten Proteine maskiert werden, wenn diese eine ähnliche Masse haben. Diesem Problem kann durch die vorherige Inkubation der fixierten Zellpellets mit goldmarkierten Antikörpern begegnet werden, welche die PCa-Marker PSMA und PAP binden. Durch die hohe Masse der Goldpartikel werden bei der MS die Signale der Antikörper auf einen Massebereich angehoben, in welchem nur ein sehr geringes Grundrauschen besteht. Hierdurch kann eine deutliche Erhöhung der Sensitivität des Verfahrens erreicht werden, sodass die PCa-Marker auch bei niedriger Abundanz quantifiziert werden können.

Quantifizierung der gewonnenen Datensätze: In dem durch dieses Beispiel beschriebenen Verfahren wird sich zunächst auf die Quantifizierung von zwei etablierten PCa-Makern konzentriert (Prostata-spezifische Membranantigen, PSMA; Prostataspezifische saure Phosphatase, PAP). Dieses Beispiel ist aber nicht beschränkend und es können andere PCa-Marker verwendet werden. Es gibt mehrere etablierte Wege, die Signalstärke der Markerproteine in der Probe zu quantifizieren.

### d) Abgleich der Ergebnisse mit etablierten Referenzwerten

Um die gewonnenen Ergebnisse einer individuellen Untersuchung bewerten zu können, können diese mit einer Referenz abgeglichen werden. Hierzu wird der Datensatz einer vorab durchgeführten klinischen Prüfung herangezogen, in welcher eine statistisch aussagekräftige Anzahl von Patienten mit dem Verfahren untersucht wurden.

Fall-Kontroll-Studie zur Etablierung von Referenzwerten: Für eine klinische Studie werden verschiedene klinische Parameter (i.e. PSA-Wert, Gleason Score, TNM-Klassifikation nach Prostatektomie, Alter) erhoben und in Relation zu den gemessenen Ergebnissen des erfindungsgemäßen Verfahrens gesetzt. Im Folgenden werden die Studienpopulation und beispielhafte Ergebnisse der Studie veranschaulicht, wobei im präsentierten Beispiel für eine bessere Verständlichkeit die Ergebnisse der Durchflusszytometrie mit dem Gleason Score aus einer Stanzbiopsie des Patienten verglichen werden. Ein anderes Beispiel lässt sich mit den Ergebnissen der alternativ beschriebenen Proteomanalyse (anstatt der Durchflusszytometrie) und unter Heranziehung anderer klinischer Parameter beschreiben (z.B. PSA-Wert, Alter, Entscheidung zur Prostatektomie, etc.).

Patientenkollektiv (Gleason Score) der Fall-Kontroll-Studie:
Kohorte 1: 100 "Low Risk" Patienten mit geringem Metastasierungsrisiko (gut differenziertes Biopsiegewebe, Gleason-Score 6).
Kohorte 2: 100 "High Risk" Patienten mit hohem Metastasierungsrisiko (schwach differenziertes Biopsiegewebe, Gleason-Score 8-9).

Die Testergebnisse am Patientenkollektiv führen zu der Etablierung von Referenzwerten (Prozentsatz von PAP+ und PSM+ Zellen; ermittelt über Durchflusszytometrie der isolierten somatischen Zellen aus Ejakulat), z.B.:

| | PAP+ | PSMA+ | PAP+ PSMA+ |
|---|---|---|---|
| "Low Risk" Patienten (n=100) | 1 % - 5 % | 2 % - 6 % | 0 % - 1 % |
| "High Risk" Patienten (n=100) | 5 % - 10 % | 6 % - 12 % | 1 % - 5 % |

### Abgleich von individuellen Testergebnissen mit etablierten Referenzwerten:

Bei einem Patienten mit Prostatakarzinom wurde im Februar 2024 eine Stanzbiopsie entnommen und die Untersuchung ergab, dass sein Prostatakarzinom indolent und relativ gut ausdifferenziert ist (Gleason 6). Vor der Stanzbiopsie hat der Patient eine Ejakulatprobe abgegeben, welche über das erfindungsgemäße Verfahren analysiert wurde. Ein Abgleich der Ergebnisse mit den etablierten Referenzwerten ergibt, dass die Probe des Patienten innerhalb des für sein Tumorstadium typischen Bereiches liegt (konkret: 2 % PAP+, 3 % PSMA+, 1 % PAP+ PSMA+). Zwei Monate später gibt der Patient während einer Kontrolluntersuchung eine weitere Ejakulatprobe ab. Die Probe liegt weiterhin innerhalb des definierten Referenzbereichs und unterscheidet sich in ihrer Zusammensetzung nicht wesentlich von der vorherigen Probe. Die Ejakulatwerte des Patienten werden weiterhin alle drei Monate kontrolliert und es finden sich über längere Zeit keine Hinweise auf eine Progression seines Prostatakarzinoms. Nach drei Jahren ändert sich jedoch die Zusammensetzung der Zellen im Ejakulat (konkret: 8 % PAP+, 12 % PSMA+, 5 % PAP+ PSMA+), obwohl sich der PSA-Wert im Blut nicht verändert hat. Aus diesem Grund wird eine weitere Biopsie angeordnet. Das Ergebnis der Biopsie zeigt, dass der Tumor mittlerweile in ein aggressiveres Stadium übergegangen ist (Gleason 8). Bei Prostatektomie werden die Lymphknoten entnommen und pathologisch untersucht. In den Lymphknoten werden keine Tumorinfiltrate gefunden, weil die Progression zeitnah auf die erhöhte Tumorzellrepräsentation im Ejakulat bemerkt wurde.

### e) Anwendung des Verfahrens in der klinischen Praxis

Anwendung des Verfahrens bei der Active Surveillance: Durch einen Abgleich der gewonnenen Werte mit den ermittelten Referenzwerten aus der klinischen Prüfung kann ein Risk-Score mit Aussagekraft darüber bestimmt werden, mit was für einer Wahrscheinlichkeit der Tumor in ein aggressiveres Stadium voranschreitet. Da es sich um ein kostengünstiges, nicht invasives diagnostisches Verfahren handelt, ist eine longitudinale Beobachtung der Tumorentwicklung möglich und vorgesehen. Im Folgenden wird der mögliche Ablauf einer solchen longitudinalen Beobachtung des Krankheitsverlaufes tabellarisch veranschaulicht:

| | Jan-25 | Mär-25 | Mai-25 | Jul-25 | Sep-25 | Nov-25 | Jan-26 | Mär-26 |
|---|---|---|---|---|---|---|---|---|
| PAP+ (%) | 2 | 3 | 6 | 2 | 6 | 5 | 12 | 14 |
| PSMA+ (%) | 5 | 4 | 3 | 5 | 8 | 7 | 16 | 13 |
| PSA+ PSMA+ (%) | 1 | 1 | 1 | 2 | 4 | 3 | 5 | 6 |
| Klinische Entscheidung | Warten | Warten | Warten | Warten | Warten | Warten | Warten | Prostatektomie |

Zur Entscheidungsfindung des Arztes können neben dem vorliegenden Test in der klinischen Praxis die Ergebnisse mehrerer verfügbarer diagnostischer Methoden integriert werden (z.B. Blut-PSA Wert, DRE, Allgemeinzustand, Biopsiebefund).

Nach der chirurgischen Entfernung der Prostata ist dieses Verfahren nicht mehr anwendbar, da keine Ejakulatprobe mehr abgegeben werden kann. Insofern schränkt dies das verfügbare Patientenkollektiv für die Methode auf Patienten vor der Entscheidung zur radikalen Prostatektomie ein.

### Anwendung des Verfahrens zur Erfolgskontrolle von Therapieansätzen exklusive der radikalen Prostatektomie:

Das Verfahren eignet sich nicht nur für die Beobachtung des PCa im Active Surveillance Ansatz, sondern auch für die Erfolgskontrolle von Therapieansätzen exklusive der radikalen Prostatektomie (z.B. Bestrahlung oder Immuntherapie). Hierbei ist eine verringerte, oder aber auch eine vermehrte Freisetzung von PCa-Zellen in das Ejakulat nach der therapeutischen Intervention möglich (z.B. durch vermehrten Zelltod oder vermehrte Phagocytose). Eine beispielhafte Entwicklung der nachgewiesenen Zellzahlen wird hier veranschaulicht:

| | Jan-26 | Mär-26 | Mai-26 | Jul-26 | Sep-26 | Nov-26 | Jan-27 | Mär-27 |
|---|---|---|---|---|---|---|---|---|
| PAP+ (%) | 12 | 14 | 12 | 30 | 6 | 5 | 2 | 3 |
| PSMA+ (%) | 16 | 13 | 16 | 55 | 8 | 7 | 5 | 4 |
| PSA+ PSMA+ (%) | 5 | 6 | 5 | 10 | 4 | 3 | 1 | 1 |
| Klinische Entscheidu ng | Warten | Warten | Bestrah lung | Warten | Warten | Warten | Warten | Warten |

Im Rahmen dieser Verlaufs- und Erfolgskontrolle therapeutischer Interventionen kann außerdem die Beobachtung von Entzündungsmarkern vorteilhaft sein, zumal die angewandten Therapieansätze den Immunstatus innerhalb des Tumors beeinflussen.

### Beispiel 2

Die Versuchsdurchführung in diesem Beispiel entspricht der Versuchsdurchführung in Beispiel 1, wobei das Isolat nicht aus Ejakulat, sondern aus Exprimaturin stammt. Die vorangehende Probenaufbereitung ist einfacher, da Exprimaturin keine Spermienzellen enthält.

### a) Probenabgabe

Probenabgabe: PCa-Patienten geben zu Hause oder alternativ während einer vorgesehenen Routineuntersuchung in der urologischen Ambulanz der behandelnden Klinik eine Exprimaturinprobe ab.

### b) Isolation des Prostatasekrets

Dann wird das Prostatasekret gewonnen. Da Flüssigbiopsien vor Fixierung degradieren, ist der beschriebene Schritt zeitsensitiv und wird bevorzugt noch am Ort der Probenabgabe durchgeführt. Die Schritte nach der Probenfixierung sind weniger zeitsensitiv, sodass danach ein Probenversand ins Zentrallabor erfolgen kann.

Kühlung der Verfahrensschritte vor Fixierung: Da die Zellen mit der Verarbeitung aus ihrem biologischen Milieu entfernt werden, sind Veränderungen von ihrem ursprünglichen Zustand in der Prostata zu erwarten. Eine Abkühlung der Probe führt zu einer Verlangsamung dieser unerwünschten Prozesse.

Verfahrensprotokoll: Die folgend beschriebene Probenaufbereitung kann über ein bereitgestelltes Versuchskit durchgeführt werden, welches die Durchführung möglichst intuitiv und fehlerfrei halten soll. Jene Lösungen des Kits, welche vor dem Fixierungsschritt eingesetzt werden, können vor ihrer Verwendung im Kühlschrank gelagert werden (4 °C):
1) Zentrifugieren der Exprimaturinprobe im 50 mL Falcon-Tube für 5 min bei 1.280 g (4 °C). Das Prostatasekret im Exprimaturin hat aufgrund seines höheren Proteingehaltes eine höhere Dichte als die Urin-Fraktion der Probe. Durch Zentrifugation findet eine Separation von Prostatasekret und dem Urin in der Probe statt;
2) Verwerfen des Überstandes (Urins), wobei das Prostatasekret im Falcon-Tube zurückbleibt;
3) Verdünnen des Prostatasekrets mit 10 mL PBS (4 °C) durch auf- und abpipettieren;
4) Zentrifugieren der Exprimaturinprobe im Falcon-Tube für 30 min bei 1.280 g (RT);
5) Verwerfen des Überstandes (Prostatasekret), wobei das Pellet nicht trockenfallen darf;
6) Resuspendieren des Zellpellets in 1 mL PFA-PBS (4 % Paraformaldehyd; PFA; RT);
7) Inkubieren der Zellsuspension für 15 min zur PFA-Fixierung (Raumtemperatur; RT);
8) Stoppen der Fixierung durch Zugabe von 40 mL PBS (4 °C) und mehrfaches Invertieren des Falcon-Tubes;
9) Zentrifugieren der Probe im Falcon-Tube für 30 min bei 1.280 g (4 °C);
10) Abnahme der verdünnten PFA-PBS Lösung, Resuspendierung des Zellpellets in 1 mL PBS (4 °C);
11) Versand der Probe (= des Isolats) ins Zentrallabor (4 °C).

### c) Analyse an dem Isolat

Die Analyse erfolgt z.B. wie in Beispiel 1.

### Beispiel 3

Die Versuchsdurchführung in diesem Beispiel entspricht der Versuchsdurchführung in Beispiel 1, wobei das Isolat nicht aus physiologisch unverändertem Ejakulat (nachfolgend "nativ" genannt), sondern aus Ejakulat nach Vasektomie des Patienten stammt (nachfolgend "Vasektomie-Ejakulat" genannt). Die vorangehende Probenaufbereitung ist einfacher, da dieses Ejakulat keine Spermienzellen oder gametische Vorläuferzellen enthält.

### a) Vasektomie

Eine Vasektomie stellt einen Grenzfall der medizinischen Intervention dar. In der Regel ist sie eine Lifestyle-Entscheidung und wird von den gesetzlichen Krankenkassen in Deutschland nicht finanziert. Eine Ausnahme wird hiervon gemacht, wenn sich ein medizinischer Grund für die Vasektomie nachweisen lässt. So wird die Vasektomie durch die Krankenkassen beispielsweise finanziert, wenn die Partnerin des Patienten aus medizinischen Gründen nicht schwanger werden darf und eine Verhütung auf anderem Wege nicht mach- oder zumutbar ist. Beim vorliegenden Verfahren kann für einen triftigen medizinischen Grund der Vasektomie argumentiert werden. Bei Männern mit Prostatakarzinom ist in der Regel kein Kinderwunsch mehr vorhanden, wodurch der Eingriff von diesem Aspekt her keine Hürde darstellen sollte. Die Vasektomie kann im Rahmen der Prostatabiopsie bei Abklärung des Verdachts auf Prostatakarzinom durchgeführt werden.

### b) Probenabgabe

Probenabgabe: Die vasektomierten PCa-Patienten geben zu Hause oder alternativ während einer vorgesehenen Routineuntersuchung eine Ejakulatprobe in der urologischen Ambulanz der behandelnden Klinik ab.

### c) Isolation von ejakulierten Zellen und Prostatasekret aus Vasektomie-Ejakulat

Im Folgeschritt werden die ejakulierten Zellen und das Prostatasekret aus der Probe isoliert. Das Prostatasekret unterscheidet sich vom Seminalplasma einer nativen Ejakulatprobe, da hier keine Beimischung aus dem Hoden stattgefunden hat. Sie ist mit dem Prostatasekret aus dem Exprimaturin nach Prostatamassage vergleichbar. Allerdingt wird in diesem Fall eine deutlich erhöhte Menge des Prostatasekrets und enthaltenen Zellen freigesetzt als beim Exprimaturin, und es findet keine Beimischung durch die Harnblase statt. Aus diesem Grund handelt es sich diagnostisch gesehen um eine höherwertigere Flüssigbiopsie mit erhöhter Menge, welche eine erhöhte Sensitivität durchgeführter Downstream-Methoden erlaubt, als wenn mit Exprimaturin gearbeitet wird.

Da Flüssigbiopsien vor Fixierung degradieren, ist der beschriebene Schritt zeitsensitiv und wird bevorzugt noch am Ort der Probenabgabe durchgeführt. Die Schritte nach der Probenfixierung sind weniger zeitsensitiv, sodass danach ein Probenversand ins Zentrallabor erfolgen kann.

Kühlung der Verfahrensschritte vor Fixierung: Da die Zellen mit der Verarbeitung aus ihrem biologischen Milieu entfernt werden, sind Veränderungen von ihrem ursprünglichen Zustand in der Prostata zu erwarten. Eine Abkühlung der Probe führt zu einer Verlangsamung dieser unerwünschten Prozesse.

Verfahrensprotokoll: Die folgend beschriebene Probenaufbereitung kann über ein bereitgestelltes Versuchskit durchgeführt werden, welches die Durchführung möglichst intuitiv und fehlerfrei halten soll. Jene Lösungen des Kits, welche vor dem Fixierungsschritt eingesetzt werden, können vor ihrer Verwendung im Kühlschrank gelagert werden (4 °C):
1) Zentrifugieren der Vasektomie-Ejakulatprobe im 15 mL Falcon-Tube für 30 min bei 1.280 g (RT).
2) Überführung des Überstandes (Prostatasekret) in ein neues 15 mL Falcon-Tube, wobei die ejakulierten Zellen als Pellet im Tube verbleiben.
3) Mit den isolierten Zellen wird nach Schritten 4-9 verfahren. Das isolierte Prostatasekret wird eingefroren (-20 °C).
4) Resuspendieren des Zellpellets in 1 mL PFA-PBS (4 % Paraformaldehyd; PFA; RT);
5) Inkubieren der Zellsuspension für 15 min zur PFA-Fixierung (Raumtemperatur; RT);
6) Stoppen der Fixierung durch Zugabe von 14 mL PBS (4 °C) und mehrfaches Invertieren des Falcon-Tubes;
7) Zentrifugieren der Probe im Falcon-Tube für 30 min bei 1.280 g (RT);
8) Abnahme der verdünnten PFA-PBS Lösung, Resuspendierung des Zellpellets in 1 mL PBS (4 °C);
9) Versand der Proben (= der Isolate) mit den isolierten Zellen (4 °C) und dem Prostatasekret (-20 °C) ins Zentrallabor.

### c) Analyse an den Isolaten

Die Analyse der isolierten Zellen erfolgt z.B. wie in Beispiel 1. Die Analyse des isolierten Prostatasekrets geschieht wie in Beispiel 1, wobei die Option der Durchflusszytometrie entfällt und bevorzugt eine HPLC-MS genutzt wird.

## Patentansprüche

1. Verfahren zur Analyse eines tumorspezifischen Markers, wobei das Verfahren umfasst:
Durchführen einer Analyse an einem Isolat, wobei ein tumorspezifischer Marker ausgewählt aus einem Protein, einer DNA und einer RNA analysiert wird, wobei das Isolat durch Fraktionieren von Ejakulat in eine Zellpopulation geringer Dichte und in eine Zellpopulation hoher Dichte erhalten wurde, wobei die Zellpopulation hoher Dichte, welche reife Spermienzellen umfasst, abgetrennt wurde und das Isolat die Zellpopulation geringer Dichte umfasst, wobei das Isolat ein Zellpellet umfasst.

2. Verfahren nach dem vorherigen Anspruch,
**dadurch gekennzeichnet, dass**
das Verfahren ein Verfahren zum Diagnostizieren und/oder Überwachen eines Prostatakarzinoms ist.

3. Verfahren nach irgendeinem der vorherigen Ansprüche,
**dadurch gekennzeichnet, dass**
das Verfahren ein Verfahren zur Erfolgskontrolle einer Therapie eines Prostatakarzinoms ist.

4. Verfahren nach irgendeinem der vorherigen Ansprüche,
**dadurch gekennzeichnet, dass**
das Verfahren das Fraktionieren von Ejakulat in eine Zellpopulation geringer Dichte und in eine Zellpopulation hoher Dichte umfasst, wobei die Zellpopulation hoher Dichte, welche reife Spermienzellen umfasst, abgetrennt wird und das Isolat die Zellpopulation geringer Dichte umfasst.

5. Verfahren nach irgendeinem der vorherigen Ansprüche,
**dadurch gekennzeichnet, dass**
das Fraktionieren von Ejakulat ein Zentrifugieren des Ejakulats umfasst.

6. Verfahren nach irgendeinem der vorherigen Ansprüche,
**dadurch gekennzeichnet, dass**
das Ejakulat vor dem Fraktionieren einer Wärmebehandlung bei mehr als 25 °C unterzogen wird.

7. Verfahren nach irgendeinem der vorherigen Ansprüche,
**dadurch gekennzeichnet, dass**
das Fraktionieren von Ejakulat ein Zentrifugieren des Ejakulats umfasst, wobei die Zellpopulation geringer Dichte aus somatischen Zellen und gametischen Vorläuferzellen geringer Dichte gebildet wird, wobei das Zentrifugieren des Ejakulats zu einer Anreicherung der somatischen Zellen in dem Isolat und einer Abtrennung reifer Spermienzellen führt, wobei zum Zentrifugieren vorzugsweise eine Zentrifugationslösung verwendet wird, wobei die Zentrifugationslösung eine Dichte aufweist, die zwischen der Dichte reifer Spermienzellen und der Dichte der somatischen Zellen und der gametischen Vorläuferzellen liegt.

8. Verfahren nach irgendeinem der vorherigen Ansprüche 5-7,
**dadurch gekennzeichnet, dass**
beim Zentrifugieren des Ejakulats ein inerter Farbstoff zur optischen Unterscheidbarkeit der Zellpopulation geringer Dichte und der Zellpopulation hoher Dichte verwendet wird.

9. Verfahren nach irgendeinem der vorherigen Ansprüche 5-8,
**dadurch gekennzeichnet, dass**
beim Zentrifugieren des Ejakulats ein Zellpellet aus den reifen Spermienzellen gebildet und abgetrennt wird.

10. Verfahren nach irgendeinem der vorherigen Ansprüche 5-9,
**dadurch gekennzeichnet, dass**
das Verfahren nach dem Fraktionieren mittels Zentrifugieren unter Verwendung einer Zentrifugationslösung und vor dem Durchführen der Analyse umfasst:
Verdünnen der Zentrifugationslösung sowie Zentrifugieren, wobei ein Zellpellet gebildet wird.

11. Verfahren nach irgendeinem der vorherigen Ansprüche,
**dadurch gekennzeichnet, dass**
das Ejakulat von einer Person stammt, bei der vor Abgabe einer Probe des Ejakulats eine Prostatamassage durchgeführt wurde.

12. Verfahren nach irgendeinem der vorherigen Ansprüche,
**dadurch gekennzeichnet, dass**
das Verfahren das Abholen einer Probe von Ejakulat an einem privaten Aufenthaltsort, vorzugsweise am Wohnort, einer Person umfasst, wobei das Fraktionieren an dem Ejakulat der Person durchgeführt wird.

13. Verfahren nach irgendeinem der vorherigen Ansprüche,
**dadurch gekennzeichnet, dass**
der tumorspezifische Marker aus prostataspezifischem Membranantigen (PSMA), prostataspezifischem Antigen (PSA), prostataspezifischer saurer Phosphatase (PAP), Prostein (SLC45A3, auch P501S), Homeobox protein Nkx-3.1 (NKX3.1), Pyruvatkinase M2, Tissue Polypeptide Antigen, Thymidinkinase, Phosphatase and tensin homolog (PTEN), Androgen-Rezeptor (AR; inklusive Amplifikationen, Mutationen und Splicevarianten), Östrogen-Rezeptoren (ESR1, ESR2), Keratinen (KRT14, KRT15, KRT18, KRT19), Kallikrein related peptidase 2 (KLK2), Schlafen 11 (SLFN11), Breast cancer type 1 and 2 susceptibility proteins (BRCA1, BRCA2), Plastin3 (PLS3), Aldehyde dehydrogenase 1A1 (ALDH1A1), Epithelial cell adhesion molecule (EPCAM), Vimentin (VIM), Cadherinen (CDH1, CDH2), Synaptophysin (SYP), Chromogranin (CD56) sowie Delta-like protein 3 (DLL3) ausgewählt ist.

14. Verfahren zumindest nach irgendeinem der vorherigen Ansprüche,
**dadurch gekennzeichnet, dass**
bei der Analyse an dem Isolat zumindest ein Marker ausgewählt aus
• Markern für Immunzell-Infiltration (CSF1, CXCL8, CXCR4, G-CSF, MCP-1, SDF-1),
• T-Zell-Differenzierungsmarkern (CD3, CD4, CD8, CD25, CD28, CD40L, CTLA4, FasL, PD-1),
• Antigen-Präsentations-Markern (MHC I, MHC II),
• B-Zell-Differenzierungsmarkern (CD19, CD20, CD21, CD40, CD80, CD86),
• Granulozyten-Differenzierungsmarkern (CD11b, CD13, CD14, CD15, CD16, CD18, CD31, CD32, CD33, CD66b, CD117, CD123, CD125, CD170, CD193, Fcε-Rezeptoren, Myeloperoxidase),
• Makrophagen-Differenzierungsmarkern (ARG1, CCR2, CD9, CD16, CD40, CD68, CD80, CD86, CD115, CD163, CD169, CD206, CD301, CSF1, CSF1R, CX3CR1, Dectin-1, F4/80, Fizz1, Lyve1, MARCO, NOS2, PDGF beta, PDL2, PPARG, TLR2, TLR4, TREM2),
• Inflammatorischen Cytokinen und Chemokinen (CCL2, CCL13, CCL18, IFN-γ, IL1a, IL1b, IL6, IL-1, IL-4, IL-5, IL-8, IL-10, IL-12, IL-13, TGF-β, TNF-α)
analysiert wird.

15. Verwendung eines Isolats aus Ejakulat, wobei das Isolat ein Zellpellet umfasst, wobei das Isolat durch Fraktionieren von Ejakulat in eine Zellpopulation geringer Dichte und in eine Zellpopulation hoher Dichte erhalten wird, wobei die Zellpopulation hoher Dichte, welche reife Spermienzellen umfasst, abgetrennt wird und das Isolat die Zellpopulation geringer Dichte umfasst, zum Diagnostizieren und/oder Überwachen eines Prostatakarzinoms und/oder zur Erfolgskontrolle einer Therapie eines Prostatakarzinoms.
